# EUROPEAN PATENT APPLICATION

(11) **EP 4 258 271 A1**
(43) Date of publication of application: **11.10.2023**
(21) Application number: 22290016.9
(22) Date of filing: 06.04.2022
(51) Int. Cl.: G16H 10/40, G01N 35/00, G06Q 10/06, G06Q 50/00, G16H 40/20, G06Q 10/10

(54) **COMPUTER-IMPLEMENTED METHOD AND SYSTEM FOR PROCESSING AND/OR TESTING OF A BIOLOGICAL SAMPLE**

(71) Applicant: Beckman Coulter, Inc., Brea, CA 92821 (US)
(72) Inventor: VARLET, Eric, 59790 Ronchin (FR); REICHLE, Roland, 80689 München (DE); RIVERS, Steven P., Minneapolis, MN 55419 (US)
(74) Representative: Müller-Boré & Partner Patentanwälte PartG mbB

(57) **Abstract**

An aspect of the present invention relates to a computer-implemented method, the method comprising at least the steps of: obtaining, by a first computing device, sample location data, the sample location data comprising information indicative of a sample location of a biological sample; obtaining, by the first computing device, test data, the test data comprising information indicative of one or more clinical tests to be performed on the biological sample; selecting by evaluating laboratory data, by the first computing device, a processing laboratory from a first plurality of laboratories, wherein the processing laboratory is located at a processing laboratory location and the processing laboratory comprises one or more laboratory instruments, the one or more laboratory instruments being configured to carry out the one or more clinical tests to be performed on the biological sample; and instructing, by the first computing device, an agent to initiate a transfer of the biological sample from the sample location to the processing laboratory location. Further aspects of the present invention relate to another computer-implemented method, a computer program product and a system.

## Description

### Technical Field

The present invention relates to the field of processing and/or testing of biological samples.

### Background

Global routing and processing of tubes containing a biological sample, such as blood, within a laboratory environment, starting at tube reception or at the collection site by the laboratory environment (e.g. after tube drawing) up to all results have been verified and the tube has been archived or disposed, is currently cumbersome, complex, time-consuming and cost intensive.

In general, the amount or number of biological samples to be analyzed and/or processed by a laboratory environment has been increased such that the reliability of test results and/or processing time may be affected negatively.

### Summary

Therefore, it is a problem to be solved by the present invention to improve reliability and efficiency of biological sample processing. Specifically, improved computer-implemented methods and an improved system are to be provided.

This problem is solved by the independent claims. Specific embodiments result from the dependent claims.

Herein, the term "biological sample" may also be referred to as "sample". The term "laboratory" may also be referred to as "lab".

A first aspect of the present invention relates to a computer-implemented method, the method comprising at least the steps of:
- obtaining, by a first computing device, sample location data, the sample location data comprising information indicative of a sample location of a biological sample;
- obtaining, by the first computing device, test data, the test data comprising information indicative of one or more clinical tests to be performed on the biological sample;
- selecting by evaluating laboratory data, by the first computing device, a processing laboratory from a first plurality of laboratories, wherein the processing laboratory is located at a processing laboratory location and the processing laboratory comprises one or more laboratory instruments, the one or more laboratory instruments being configured to carry out the one or more clinical tests to be performed on the biological sample; and
- instructing, by the first computing device, an agent to initiate a transfer of the biological sample from the sample location to the processing laboratory location.

Specifically, the steps may be carried out subsequently and without interruption. In particular, the step of instructing an agent may be carried out subsequently to selecting the processing laboratory autonomously by the computing device. That is, the step of instructing an agent may be carried out subsequently to selecting the processing laboratory by the computing device in an automatic fashion without any intervention of e.g. a user being necessary.

Further advantageously, this aspect of the present invention allows for reducing processing time, including transportation time of a sample, and/or transportation distance of a sample between laboratories of a plurality of laboratories, for example within a laboratory environment. The above-mentioned aspect may allow for reducing contamination risks and the possibility of going out of the reliability window of the sample, thereby improving the reliability of the sample test results. In addition, the workload of the laboratories of the plurality of laboratories can be balanced. Thus, wasting of processing-bandwidth, i.e. wasting of the capacity of one or more processing laboratories of processing samples, or wasting laboratory resources, such as available laboratory instruments, of the first plurality of laboratories is avoided while at the same time the processing time required for clinical tests to be performed on the biological sample may not increase. Although the complete capacity of one or more processing laboratories may be used, the processing time may not change, in particular increase. The processing time may be tuned with predetermined constraints comprising, for example, the time window within which a sample provides reliable results, i.e. the stability period of the sample. Furthermore, the transportation time can be tuned with predetermined time constraints of the sample and of the tests to be carried out on the sample. The time constraints may comprise the time window within which the sample provides reliable results or the minimum amount of time that shall occur between a pre-analytical step, such as centrifuging, mixing with a reagent, etc., and the tests. Also, transportation distance may be tuned such that potential risks of contamination, for example due to environmental factors, may be reduced. A sample may be routed, in particular transferred or transported, from lab site to lab site within a lab environment according to the current or future state of the lab sites.

Moreover, lab-resources may be used more efficiently. That is, on the one hand, queuing times of samples until they get processed and, on the other hand, idle times of laboratory instruments which are currently not used or working can be reduced. Samples to be handled or processed can be routed or distributed to the next, timely and/or spatially, available laboratory and available laboratory instrument or the next, timely and/or spatially, available lab instrument within a laboratory.

In a suitable laboratory environment comprising, for example, an automated laboratory system and sample transportation means, such as conveyor bands and/or means for pneumatic delivery, sample processing may be performed autonomously.

A lab environment may comprise a plurality of laboratories. The plurality of laboratories may be contained in the lab environment.

The method may in particular be for global or lab-wide routing and processing of the sample.

The processing time of a sample is in particular an amount of time needed to handle the sample, i.e. the time needed to carry out all the tests to be performed on the sample. In particular, the processing time of a sample may be a prediction and/or an estimate of said amount of time. In particular, the processing time of the sample is given by the processing time associated to the processing laboratory. If the sample has to undergo the tests specified by the test data as well as further test, the further test being carried out by a further processing laboratory, the processing time may include the processing time associated to the processing laboratory, the amount of time needed by the further processing laboratory to carry out the further tests and the amount of time needed to transfer the sample from the processing laboratory and the further processing laboratory.

The obtaining may comprise one or more of: determining, computing, receiving, or accessing. In the present disclosure, "obtaining, by a computing device (e.g. the first computing device), data" may comprise accessing the data.

Alternatively, "obtaining, by a computing device, data" may comprise generating the data, i.e. creating the data based on one or more inputs. In yet another example, "obtaining, by a computing device, data" may comprise accessing a first portion of the data and generating a second portion of the data from the first portion of the data.

In the present disclosure, "accessing, by a computing device (e.g. the first computing device), data" may comprise retrieving the data e.g. from the at least one memory of said computing device, from the memory of another computing device, or from another remote data storage (a database, a secondary memory, a cloud storage or the like). Accordingly, in some cases, retrieving data may comprise downloading data. Exemplarily, said computing device may retrieve data in response to a user command and/or to a notification sent by another computing device. Additionally or alternatively, "accessing, by a computing device, data" may comprise receiving the data, e.g. from a user or another computing device. The two options are not mutually exclusive. For instance, accessing, by a computing device, data may comprise receiving the data, storing the data in the memory of said computer device and retrieving the data by accessing said memory.

The first computing device may comprise or be a cloud computing system or platform.

It may comprise or be a distributed computer system. It may be a server. The first computing device may comprise or be a network of connected desktop PCs and/or workstations and/or laptops and/or tablet PCs. The network may be a LAN, e.g. a wireless LAN, a WAN, or the internet. The first computing device may be located at the sample location. The first computing device may be a desktop PC, a workstation, a laptop or a tablet PC. As described herein, a computing device, e.g. the first computing device, may comprise one or more processing units (e.g. a CPU, a GPU, or the like) and a memory.

The sample location data may comprise information specifying the location of the sample. The sample location data may comprise or consist of data containing GPS data and/or coordinates of the location of a sample. Alternatively, or in conjunction with the above, the sample location data may comprise information specifying the laboratory of the plurality of laboratories the sample is located in. For instance, the latter information may be comprised in an alphanumeric string, e.g. an identification code or number, identifying the laboratory of the plurality of laboratories the sample is located in. The sample location data may be stored in a computer-readable file. The content of said file may also be accessible, readable, and/or intelligible to a human operator.

The test data may comprise information specifying one or more clinical tests to be carried out on the biological sample. The information may include names and/or descriptions and/or identification numbers of said clinical tests. The test data may be stored in a computer-readable file. The content of said file may also be accessible, readable, and/or intelligible to a human operator. For example, the file storing the test data may be encrypted.

Both the sample location data and the test data may be stored in the same file or in at least two different files. The test data and sample location data may be received at the same time in the same file, so that the steps of obtaining said data may be the same step. Alternatively, test and/or location data may be stored in a memory, e.g. a distributed memory cache, and/or a database.

The biological sample may be a sample of a bodily fluid of a subject. For example, the bodily fluid may be a physiological fluid, such as blood, saliva, urine, sweat, amniotic fluid, cerebrospinal fluid, ascites fluid, or the like. The biological sample may comprise one or more components that may potentially comprise analytes of interest for performing the at least one clinical test. A clinical test may comprise one or more procedures that, when carried out on the biological sample or on a component thereof, allow for estimating the value of a parameter, e.g. a clinical parameter. In particular, a clinical test may comprise a physical, a biological, an optical, a mechanical, immunological, and/or chemical procedure.

Selecting the processing lab may include choosing, by the first computing device, a processing laboratory from a plurality of laboratories based on the laboratory data. The first computing device may select or choose several processing laboratories from the first plurality of labs, each of the several processing laboratories comprising different lab instruments for carrying out different clinical tests or different steps of a particular clinical test.

The selecting is done by evaluating laboratory data (hereinafter also referred to as: "lab data"). The first computing device may receive or obtain or collect the lab data from the plurality of laboratories or labs. The lab data may be obtained or received collected in one file or a set of files, for example as a data stream from each lab of the plurality of labs. The first computing device may evaluate the lab data. The evaluating may include analysing the lab data based on a set of predefined or predetermined lab selection constraints. Said set of predefined or predetermined lab selection constraints may comprise one or more constraints on the transportation time of the sample, one or more constraints on the transportation distance of the sample, and/or one or more constraints on the expected processing time of the sample. The set of e lab selection constraints may also include a threshold for the delivery time and/or transportation distance and/or expected processing time of the sample. The threshold may be set by a human operator. The evaluating may include optimizing the lab data. i.e. finding the optimal processing lab based on the lab data. The optimization of the lab data may be done by the first computing device using any suitable optimization algorithm, such as machine learning models, linear regression algorithms, basic heuristics.

The processing lab may be the lab of the plurality of labs being selected to perform or carry out one or more clinical tests on the sample. The processing lab may be a lab complex comprising several labs, wherein each lab has a plurality of lab instruments which may be distributed in different labs located close to one another in the lab complex.

The one or more lab instruments may comprise one or more standalone instruments, The one or more lab instruments may also comprise a lab automation system (hereinafter also referred to as "automated lab system").

The one or more lab instruments may comprise a robotic aiding device, such as a robotic arm, and/or conveying means to convey a sample and/or transfer the sample from an instrument to another instrument, e.g. from a standalone instrument to an automated lab system.

In an embodiment of the invention, a lab instrument may be a pre-analytical instrument, a post-analytical instrument or an analytical instrument.

Generally, an automated laboratory system (hereinafter also referred to as: "ALS") is an assembly comprising a plurality of components and a computing device, wherein the computing device is operatively connected to these components and is configured to control each component. A component may be an analytic instrument, a pre-analytic instrument, a post-analytic instrument, an input/output module, a transportation component (e.g. track, belt, tube carrier) configured to move a sample.

An analytic instrument (hereinafter also referred to as "analyser") is an instrument configured to carry out one or more analytic steps, such as measuring one or more characteristics of the sample, e.g. the concentration of a given analyte. A pre-analytic instrument is an instrument configured to carry out one or more pre-analytic steps on a biological sample to prepare said sample for the analytic instrument(s). For example, centrifuges, aliquoters and de-cappers are pre-analytic instruments. A post-analytic instrument is an instrument configured to carry out one or more post-analytic steps on the biological sample after the sample has been processed by one or more analytic instruments. For example, re-cappers and sample storage units are post-analytic instruments.

The processing lab location may be the location of a plurality of labs close to one another, e.g. the location of a plurality of instruments which, e.g. may be distributed in different labs located close to one another. The processing lab location may also be the location of a lab complex. The processing lab location may be the location of one processing lab of the plurality of labs.,

The selection of a processing laboratory by evaluating said lab data, by the first computing device, allows for shortening processing times of samples. Clinical tests performed or carried out on a sample may be carried out more efficiently.

Instructing the agent to initiate the transfer of the biological sample from the sample location to the processing laboratory location may, for instance, comprise sending and/or transferring instructions to the agent and/or displaying said instructions to the agent. In particular, the transferring instructions specify that the agent shall initiate the transferring, e.g. transfer, the biological sample from the sample location to the processing laboratory location. In particular, the instructions may comprise or consist of the sample location data associated to the processing lab location.

For example, transferring instructions may specify that the agent should move the sample to a given rack, wherein the rack s scheduled to be transferred to the processing laboratory. In this case, for instance, the transferring instructions comprise or consist of information specifying the rack into which the sample is to be inserted.

For instance, the agent may be a user. If this is the case and if the first computing device is located at the sample location, the initiating the transfer may be carried out by displaying transferring instructions to an output device, such as a screen, of the first computing device, the output device being accessible by the user. The initiating the transfer may be carried out by sending instructions to a second computing device. The second computing device may be configured to instruct, e.g. by using an I/O device, an agent to handle and prepare the sample for transport to the processing lab location. The second computing device may comprise or be a server. The second computing device may comprise or be a network of connected desktop PCs and/or workstations and/or laptops and/or tablet PCs. The network may be a LAN and/or a wireless LAN. The second computing device may be located at the sample location. The first computing device may be a desktop PC, a workstation, a laptop or a tablet PC.

The agent may be a sample handling device and, in particular, an intelligent or smart sample handling device which comprises the second computing device. The agent may comprise or be the second computing device. In this case, in particular, the sample handling device may comprise transferring means (e.g. a robotic arm) configured to initiate the transfer of the biological sample from the sample location to the processing laboratory location. For example, the sample handling device is configured to receive transferring instructions and to use the transferring instructions to insert the biological sample into a rack that is destined to be shipped to the processing laboratory.

In an embodiment, the above-described method may be performed in the following order: In a first step, the sample location data is obtained. In a next step or a step performed simultaneously with the first step, the test data is obtained. In a next step, the selection of a processing lab of a plurality of labs is done by evaluating the lab data. In a next step, in particular a final step, the agent is instructed to initiate the transfer of the sample from the sample location to the processing lab location.

In a specific embodiment, the method further comprises:
obtaining, by the first computing device, the laboratory data, the laboratory data comprising, for each laboratory of the first plurality of laboratories, information indicative of:
   a respective distance associated to said each laboratory, and/or
   a respective delivery time associated to said each laboratory, and/or
   a respective processing time associated to said each laboratory, and/or
   a respective state of said each laboratory, and/or
   a respective workload of said each laboratory and/or
   whether said laboratory is able to carry out the one or more clinical tests to be performed on the biological sample;
wherein:
   the respective distance associated to said each laboratory is a distance between the sample location and a laboratory location of said each laboratory;
   the respective delivery time associated to said each laboratory is an amount of time needed to deliver the biological sample from the sample location to the laboratory location of said each laboratory or an estimate and/or prediction of the time needed to deliver the biological sample from the sample location to the laboratory location of said each laboratory; and
   the respective processing time associated to said each laboratory is an amount of time needed to carry out the tests specified by the test data by using said each laboratory or the respective processing time associated to said each laboratory is an estimate and/or a prediction of the amount of time needed to carry out the tests specified by the test data by using said each laboratory.

Advantageously, by obtaining the lab data, the first computing device may efficiently select without any intervention by a user or by an agent the optimal processing lab with respect to the respective processing time and/or distance and/or delivery time, or the processing lab chosen based on one or more predetermined constraints. For example, the delivery time may be tuned with respect to the time window within which reliable results of the samples to be processed are provided. This may even further reduce the processing time of a sample since the lab data are evaluated, in particular analysed or optimised, with respect to in particular distance and/or delivery time from the sample location to the processing lab and/or processing time of the processing lab. Thus, unnecessary use or waste of processing bandwidth of the first plurality of laboratories is avoided.

In particular, information indicative of a state of a laboratory comprises information indicative of a state of at least an instrument of the laboratory, e.g. of the one or more instruments comprised in said laboratory, namely of a current or expected state. In other words, information indicative of a state of a laboratory may comprise information specifying a current or expected state of at least an instrument of said laboratory. The state of a laboratory may be defined by one or more aspects of the laboratory, e.g. including the states and/or functionalities of the instruments comprised in said laboratory.

Exemplarily, information indicative of a state of a laboratory comprises information indicative of the suitability of the laboratory to carry out the clinical tests to be performed on the biological sample. The information may be provided at a coarse level, e.g. for the laboratory as a whole and not the specific instruments thereof. In other words, while the suitability of a laboratory may exemplarily be determined based on the suitability of the instruments of said laboratory, the information indicative of the suitability may specify the suitability of the laboratory as a whole, without disclosing the suitability of the single components.

In particular, the suitability of a laboratory may be determined by its capability of performing the clinical tests to be performed on the biological sample and/or its availability to process a sample in view of the current and/or expected workload at the laboratory. For example, information indicative of the suitability of the instruments of a laboratory comprises information indicative of the capability of the instruments of said laboratory of performing the clinical tests and/or information indicative of the availability, e.g. of the workload, of said laboratory to process a sample, e.g. the biological sample.

In particular, the capability of a laboratory may be based on any of the following or combinations thereof: whether said laboratory comprises instruments that are configured to carry out the clinical tests to be performed on the biological sample, whether such instruments are currently functioning or not, whether the instruments of said laboratory are capable of handling a given type of container, performance characteristics of the instruments of said laboratory, such as speed or accuracy of analysis.

With regards to the availability of a laboratory, the workload may be determined based at least on 1) a number of samples being processed by said laboratory at a given timepoint, or at a given time interval and, optionally, further based on 2) a forecasted leftover time for each of these samples. In particular, the number of samples being processed by said laboratory may be a relative quantity considered with respect to a capacity of said laboratory.

In particular, the current workload may be determined based on the number of samples that are being processed by the laboratory at the moment in which the state data are accessed (e.g. the workload may be determined on-the-fly) and, optionally, their forecasted leftover time. The expected workload may be determined based on the number of samples that are anticipated to be processed by the laboratory at the moment in which the sample is expected to arrive at said laboratory (or "expected arrival time"). The anticipated number of samples may be extracted from a schedule of the laboratory, which may be accessed by the first computing device.

The information indicative of the state of a laboratory may include information specifying the state of the quality control of said instruments of the lab and/or the calibration state of said instruments of the lab. Alternatively or in conjunction with the above, information indicative of the state of a laboratory may include information specifying the amount of the reagents available to the instruments of said laboratory. The state may be an expected state of the lab. Alternatively or in conjunction with the above, information indicative of the state of a laboratory may include information specifying the availabliilty of one or more of instruments of said laboratory due to planned maintenance, e.g. in a given period of time.

Herein, the handling time associated to a laboratory is in particular an amount of time needed by said laboratory to handle the sample, i.e. the amount of time needed by said laboratory to carry out the tests specified in the test data. For instance, the handling time associated to a laboratory is an estimate and/or prediction of said amount of time. For example, the handling time associated to a laboratory may be the time interval between a first timepoint and a second timepoint. The handling time associated to a laboratory may be an estimate and/or a prediction of said time interval. For instance, the first timepoint is the timepoint at which said laboratory receives the sample, the timepoint at which the sample arrives at said laboratory, and/or the timepoint at which the arrival of the sample at the laboratory is registered and/or detected. The second timepoint may be the timepoint at which all the tests specified in the test data have been carried out by the instruments of said laboratory, the timepoint at which the sample is stored in a storage unit of said laboratory, and/or the timepoint at which the sample is disposed in a disposal unit of said laboratory.

The delivery time associated to a laboratory is in particular an amount of time needed to deliver the biological sample from the sample location to the laboratory location of said laboratory. For instance, the delivery time associated to a laboratory is a prediction and/or an estimate of said amount of time. For example, the delivery time associated to a laboratory may be the time interval between a third timepoint and the second timepoint. The delivery time associated to a laboratory may be an estimate and/or a prediction of said time interval. For instance, the third timepoint is the timepoint at which the sample leaves the sample location, the timepoint at which the sample is inserted in a rack that is destined to be shipped to said laboratory, and/or the timepoint at which said processing laboratory is selected as processing laboratory.

The processing time associated to a laboratory is the amount of time needed to carry out the tests specified by the test data by using said laboratory. In particular, the processing time associated to a laboratory is an estimate and/or a prediction of the amount of time needed to carry out the tests specified by the test data by using said laboratory. In particular, the processing time associated to a laboratory may be the time interval between a fourth timepoint and the second timepoint. The processing time associated to a laboratory may be an estimate and/or a prediction of said time interval. For example, the fourth timepoint is the (estimated) timepoint at which the sample is drawn, the (expected or estimated) timepoint at which the sample is registered at the sample location, and/or the (expected and/or estimated) timepoint at which the arrival of the sample at the sample location is registered and/or detected.

In particular, the processing time associated to a laboratory includes the delivery time associated to the laboratory and the handling time associated to the laboratory. The processing time associated to a laboratory may also include the selection time. In particular, the selection time is the amount of time needed to select the processing laboratory. For instance, the selection time is an estimate and/or a prediction of the amount of time needed to select the processing laboratory. For example, the processing time associated to a laboratory may be the time interval between the fourth timepoint and the third timepoint. The processing time associated to a laboratory may be an estimate and/or a prediction of said time interval.

For example, the processing time associated to a laboratory may be an estimate and/or prediction of the amount of time needed to carry out the tests specified by the test data by using said laboratory. Said estimate and/or prediction may be given by the delivery time associated with said laboratory, e.g. if the handling time and the selection time is much smaller than the delivery time or may be the sum of the delivery time and the handling time associated to said laboratory, e.g. if the selection time is much smaller than the delivery time and/or handling time.

The distance associated to a laboratory may be the linear distance between said laboratory and the sample location points. The distance associated to a laboratory may also be the distance between said laboratory and the sample location following a specific path, the specific path being comprised in a road netweork and connecting said laboratory to the sample location. For instance, the distance associated to a laboratory may also be the distance between said laboratory and the sample location following a specific path over an intermediate location. In other words, in this case, the distance associated to a lab may be the length of the polygonal chain connecting said lab with the sample location over the intermediate location.

Exemplarily, obtaining laboratory data may comprise obtaining from each lab of the plurality of labs a respective portion of the lab data. For each lab of the plurality of laboratories, the respective portion of lab data may comprise information indicative of the respective distance associated to said each laboratory, and/or the respective delivery time associated to said each laboratory, and/or the respective handling time associated to said each laboratory, and/or the respective processing time associated to said each laboratory and/or the respective state of said each laboratory, and/or the respective workload of said each laboratory.

Obtaining lab data may comprise, for each lab of the plurality of laboratories, generating information indicative of the respective distance associated to said each laboratory, and/or the respective delivery time associated to said each laboratory, and/or the respective handling time associated to said each laboratory, and/or the respective processing time associated to said each laboratory and/or the respective state of said each laboratory, and/or the respective workload of said each laboratory. For instance, obtaining lab data may comprise receiving from each laboratory of the plurality of laboratories information indicative of the respective handling time, obtainining, for each lab of the plurality of labs, the location of said each lab, use sais locations to calculate, for each lab of the plurality of labs, the respective distance associated to said each laboratory, and estimating the processing time associated to said each laboratory by using the respective handling time and the respective distance. For instance, for each lab of the plurality of labs, the first computing device may use the respective distance associated to said each lab to estimate the respective delivery time and may estimate the respective processing time by summing the respective handling time and the respective delivery time.

In particular, the step of selecting the processing laboratory comprises, in particular automatically by the first computing device, determining at least a first laboratory of the first plurality of laboratories so that the first laboratory fulfils one or more instruments requirements, and/or one or more distance requirements and/or one or more delivery time requirements, and/or one or more processing time requirements.

Worded differently, the step of selecting the processing laboratory comprises, in particular automatically by the first computing device, determining at least a first laboratory of the first plurality of laboratories so that a set of laboratory instruments comprised in the first laboratory fulfils one or more instruments requirements, and/or the distance associated to the first laboratory fulfils one or more distance requirements and/or the delivery time associated to the first laboratory fulfils one or more delivery time requirements, and/or the processing time associated to the first laboratory fulfils one or more processing time requirements.

Advantageously, by determining at least a first laboratory of the first plurality of laboratories on the basis of predetermined constraints such as instrument requirements and/or distance requirements and/or delivery time requirements and/or processing time requirements, the selection of a processing laboratory can be made more efficient since the evaluation of the lab data may be accelerated, specifically, since no interaction of the first computing device with any user or agent is necessary. As a result, the total processing time of a sample may be shortened and the efficiency of processing samples may be increased.

The requirements may be associated to a pre-set threshold. The threshold may for example define the number and/or type of lab instruments to carry out the clinical tests and/or the uppermost delivery time and/or the uppermost distance to a processing lab and/or the uppermost processing time of a sample.

In particular, the one or more processing time requirements comprises the requirement that the processing time associated to the first laboratory is smaller than or equal to a stability time. Alternatively or in conjunction with the above, the one or more processing time requirements comprises the requirement that the processing time associated to the first laboratory is smaller than or equal to the stability time. The stability time of a biological sample is in particular the amount of time after the drawing of the biological sample in which the biological sample may be reliably analysed. The stability time may be an estimate and/or a prediction of said amount of time. For instance, stability time is the amount of time after the drawing of the biological sample in which the one or more clinical tests to be performed on the biological sample provide reliable results. In particular, the stability time may be an estimate and/or an estimate of said amount of time. For example, the stability time associated to a laboratory may be the time interval between the timepoint at which the sample is drawn and a fifth timepoint. The fifth timepoint may be the timepoint after which the biological sample may be not reliably analysed, the timepoint after which at least one test of the tests specified by the test data does not provide reliable results. This way, the accuracy of the results of the analyses carried out on the sample is increased.

In addition to the preceding embodiment, the one or more distance requirements may comprise the requirement that the distance associated to the first laboratory is lower than or equal to a distance threshold. Additionally or alternatively, the one or more distance requirements may comprise the requirement that, for each laboratory of a second plurality of laboratories, the distance associated to the first laboratory is smaller than or equal to the distance associated to said each laboratory.

Additionally or alternatively, the one or more instruments requirements may comprise the requirement that the set of laboratory instruments comprised in the first laboratory is configured to carry out the one or more clinical tests.

Additionally or alternatively, the one or more delivery time requirements may comprise the requirement that the delivery time associated to the first laboratory is lower than or equal to a time delivery threshold. Additionally or alternatively, the one or more delivery time requirements may comprise the requirement that, for each laboratory of the plurality of laboratories, the delivery time associated to the first laboratory is smaller than or equal to the delivery time associated to said each laboratory.

Additionally or alternatively, the one or more processing time requirements may comprise the requirement that the processing time associated to the first laboratory is lower than or equal to a processing time threshold. Additionally or alternatively, the one or more processing time requirements may comprise the requirement that, for each laboratory of the second plurality of laboratories, the processing time associated to the first laboratory is smaller than or equal to the processing time associated to said each laboratory.

Additionally or alternatively, in order for the first computing device to select the first laboratory as processing laboratory, the first laboratory may be to fulfil the one or more instruments requirements, and/or the one or more distance requirements, and/or the one or more delivery time requirements, and/or the one or more processing time requirements, wherein the first plurality of laboratories may comprise the second plurality of laboratories.

Advantageously, by choosing the requirements, in particular the thresholds, as described above, the processing time of a sample may be reduced. In particular, the selection of the processing laboratory by the first computing device may be accelerated. That is, the efficiency of processing samples may be increased further.

In an embodiment, the second plurality of laboratories is equal to the first plurality of laboratories. In particular, each laboratory of the first plurality of laboratories may be comprised in the second plurality of laboratories. In a further embodiment, the second plurality of laboratories is a subset of the first plurality of laboratories, in particular a proper subset of the first plurality of laboratories. In a further embodiment, the second plurality of laboratories may comprise or consist of the laboratories of the first plurality of laboratories that fulfil the instruments requirements. In particular, the step of selecting the processing laboratory may comprise generating the second plurality of laboratories by selecting, among the first plurality of laboratory instruments, the laboratories that fulfil the instruments requirements.

Exemplarily, the selecting of the processing laboratory comprises determining whether the first laboratory fulfils a set of selection requirements and, if the first laboratory fulfils the selection requirements, selecting the first laboratory as processing laboratory. In particular, the set of selection requirements comprise the one or more instruments requirements, and/or the one or more distance requirements, and/or the one or more delivery time requirements, and/or the one or more processing time requirements.

In a specific embodiment, the method may further comprise the steps of:
determining, by the first computing device, a first processing step to be carried out on the biological sample at the sample location; and
instructing, by the first computing device, the agent, in particular the second computing device, to initiate the carrying out of the first processing step on the biological sample at the sample location.

Exemplarily, the step of determining the first processing step is based on processing laboratory data, the processing laboratory data comprising information indicative of a state and/or a configuration of the one or more laboratory instruments of the processing laboratory.

Alternatively, or in conjunction with the above, the step of determining the first processing step may be based on the laboratory data. In particular, the step of determining the first processing step is based on the respective distance associated to the processing laboratory, and/or the respective delivery time associated to the processing laboratory, and/or the respective handling time associated to the processing laboratory and/or the respective processing time associated to the processing laboratory.

Alternatively, or in conjunction with the above, the step of determining the first processing step may be based on processing data, the processing data comprising information indicative of one or more processing steps that can be carried out at the sample location. In particular, the method according to the first aspect of the present invention may comprise the step of obtaining, by the first computing device, the processing data.

Advantageously, according to this embodiment, the processing of a sample can be rendered even faster and less resource-consuming, i.e. without wasting lab capacities thus without wasting processing bandwidth of the first plurality of laboratories. Idle times of laboratories, in particular laboratory instruments, can be reduced further. A sample may be handled or processed iteratively until all processing steps, for example included in one or more tests to be carried out on the sample, are completed. Sample processing can be done more efficiently and autonomously further lowering the risk of spoiled samples and/or unreliable results.

The determining of a first processing step may be based on processing lab data. That is, the first computing device may evaluate or analyse, in particular optimise, the processing laboratory data with respect to the state and/or the configuration of the lab instruments of the processing lab. The state may comprise the occupation of one or more lab instruments for carrying out a particular processing step or clinical test or the availability of one or more lab instruments to carry out a particular processing step or clinical test. The state may also be defined as described above. The configuration may comprise whether the one or more lab instruments of the processing lab are configured to carry out the particular processing step or clinical test. The configuration may also comprise whether the processing lab is equipped with the lab instruments to carry out the particular processing step or clinical test.

In an embodiment, a number of labs, in particular all labs, of the plurality of labs that fulfils the instruments requirements, and/or the delivery time requirements, and/or the distance requirements may be determined. One of these labs may be picked based on one or more selection criteria, such as the one with minimum delivery time or distance.

The determining of the first processing step may be based on the processing data. In particular, the first computing device may determine, by using the test data, the one or more processing steps needed to carry out the one or more tests. In particular, the first computing device may assess whether at least a processing step is associated to a settling time and select a processing step associated to a settling time as first processing step. The settling time associated to a processing step is in particular an amount of time after the completion of the first processing step that is to be waited before carrying out further processing steps. If this is the case, the first computing device may access processing labs and assess whether the first processing step may be carried at the sample location. If this is the case, the first processing device may instruct, the agent, in particular to initiate the carrying out of the first processing step on the biological sample at the sample location and to initiate the transfer of the sample after completion of the processing step. In this case, the settling time may be used for transporting the biological sample, thereby reducing the processing time of the sample. Instructing the agent to initiate the carrying out of the first processing step on the biological sample may, for instance, comprise sending and/or transferring processing instructions to the agent and/or displaying said instructions to the agent. In particular, the processing instructions instruct the agent, e.g. the second computing device, to initiate the carrying out of the first processing step on the biological sample.

If the agent is a user and if the first computing device is located at the sample location, the initiating of the carrying out of the first processing step may be performed by displaying the processing instructions to an output device accessible by the user. The initiating of the carrying out may be performed by sending processing instructions to a second computing device. The second computing device may be configured to instruct, e.g. by using an I/O device, an agent to prepare the sample for carrying out the first processing step.

The agent may be a sample-handling device comprising the second computing device and transferring means (e.g. a robotic arm) configured to initiate the carrying out of the first processing step transfer of the biological sample from the sample location to the processing laboratory location. For example, the sample-handling device is configured to receive processing instructions and to use said instructions to insert the biological sample into a input module of an instrument configured to carry out the first processing step.

The processing lab data may comprise information indicative of the state of the processing laboratory and, in particular, the information indicative of the suitability of the laboratory to carry out the clinical tests to be performed on the biological sample. The information may also be indicative of the state of the lab instruments or other processing devices which are located at the sample location and can be used to process the sample.

In addition to the preceding embodiment, the method may further comprise the step of:
obtaining, by the first computing device, processing data, the processing data comprising information indicative of one or more processing steps that can be carried out at the sample location,
wherein the step of determining the first processing step is further based on the processing data.

Advantageously, this allows for streamlining the sample processing and thus saves time. For example, a reagent that needs time to react with the sample may be included and then the sample may be transported. In this way, the reaction time of the reagent may be included in the transport time and, hence, time can be saved. Thereby, the advantages and/or improvements of the preceding embodiment may be enhanced. Specifically, the processing bandwidth of the first plurality of laboratories is conserved.

The information indicative of the processing steps that can be carried out at the sample location may include information indicative of the state, for example, the availability and/or configuration and/or workload, of at least a subset of the lab instruments available at the sample location.

In particular, according to the present disclosure, information indicative of a state of a subset of lab instruments comprises information specifying a current or expected state of each lab instrument of said subset. The information indicative of the state of a lab instrument may include information specifying the state of the quality control of said instrument and/or the calibration state of said intrument and/or information specifying the amount of the reagents available to said instrument of said laboratory. Exemplarily, according to the present invention, information indicative of a state of a laboratory instrument comprises information indicative of the suitability of said instrument to carry out the clinical tests to be performed on the biological sample. In particular, the suitability of a lab instrument may be determined by its capability of performing processing steps, e.g. the first processing step, on the biological sample and/or its availability to perform processing steps, e.g. the first processing step, in view of the current and/or expected workload of the lab instrument. In particular, the capability of a lab instrument may be based on any of the following or combinations thereof: whether said lab instrument is configured carry out the clinical tests to be performed on the biological sample, whether said instrument is currently functioning or not, whether said instrument is capable of handling a given type of container, performance characteristics of said instrument, such as speed or accuracy of analysis. With regards to the availability of a laboratory instrument, the workload may be determined based at least on 1) a number of samples being processed by said instrument at a given timepoint and, optionally, further based on 2) a forecasted leftover time for each of these samples. In particular, the number of samples being processed by said instrument may be a relative quantity considered with respect to a capacity of said instrument. In particular, the current workload may be determined based on the number of samples that are being processed by the instrument at the moment in which the state data are accessed (e.g. the workload may be determined on-the-fly) and, optionally, their forecasted leftover time. The expected workload may be determined based on the number of samples that are anticipated to be processed by the lab instrument at the moment in which the sample is expected to arrive at said laboratory instrument. The anticipated number of samples may be extracted from a schedule of the laboratory, which may be accessed by the first computing device.

In an embodiment, the step of selecting the processing laboratory may comprise optimising, by using the laboratory data, a delivery time of the biological sample and/or a processing time of the biological sample and/or a processing time of the biological sample and/or or a distance from the sample location.

In an embodiment, all the labs that fulfil the instruments requirements may be determined, and, among these labs, the delivery time and/or the distance may be optimised.

In an embodiment, in order for the first computing device to select, e.g. autonomously select, the first laboratory as processing laboratory, for each laboratory of the second plurality of laboratories, the distance associated to said each laboratory is to be greater than or equal to the distance associated to the first laboratory.

The optimising of the distance may comprise or consist of: determining a second laboratory of the first plurality of laboratories such that, for each laboratory of the second plurality of laboratories, the distance associated to said each laboratory is greater than or equal to the distance associated to the second laboratory, and selecting the second laboratory as processing laboratory.

In an embodiment, in order for the first computing device to select the first laboratory as processing laboratory, for each laboratory of the second plurality of laboratories, the delivery time associated to said each is to be greater than or equal to the delivery time associated to the first laboratory.

In an embodiment, in order for the first computing device to select the first laboratory as processing laboratory, for each laboratory of the second plurality of laboratories, the processing time associated to said each laboratory is to be greater than or equal to the processing time associated to the first laboratory.

The optimising of the delivery time may comprise or consist of: determining a third laboratory of the plurality of laboratories such that, for each laboratory of the second plurality of laboratories, the delivery time and/or the processing time and/or the handling time associated to said each laboratory is greater than or equal to the delivery time or the processing time or the handling time associated to the third laboratory, respectively, and selecting the third laboratory as processing laboratory.

Advantageously, by the above-described optimisation of lab data, samples may be processed even more efficiently. In particular, processing times may be reduced even further and processing the samples may be streamlined even further. By optimising the lab data, the selection of a processing lab is improved, rendering the selection particularly more reliable and efficient. Thus, laboratory resources may be used even more efficiently. Consequently, samples may be processed even more reliably and efficiently. In particular, the advantages described above with respect to the first plurality of laboratories equally applies with respect to the second plurality of laboratories.

In an embodiment, the selecting of the processing lab may include: determining the processing steps, such as centrifugation, necessity to carry out a test, determining the number of tubes in or planned or expected or estimated to be in a first lab and a second lab that have to undergo the processing steps, evaluating the processing time associated to the first lab by using the handling time and the delivery time associated to the first lab, evaluating the processing time associated to the first lab by using the handling time and the delivery time associated to the second lab. In particular, the handling time associated to the first lab and the handling time associated to the second lab are evaluated by using the number of tubes expected to be processed by the first lab and the second lab, respectively. The selecting of the processing lab comprises choosing, among the first and second lab, the lab with the smallest processing time.

In a further embodiment, the selecting may include: determining, in particular autonomously determining, by the first computing device, the processing steps, such as centrifugation, needed to carry out more than one test, determining the number of tubes in or planned or expected or estimated to be in a first lab and a second lab that have to undergo the processing steps, evaluating the handling time associated to the first lab and the handling time associated to the second lab, and selecting, among the first and the second lab, the lab with shortest processing time as processing laboratory. In particular, the handling time associated to a laboratory may be obtained by evaluating, for each test to be carried out on the sample, a respective time needed by the laboratory to carry out said test and by obtaining the handling time as the sum of those respective times evaluated as described above. Optionally, the delivery time from the first lab to the second lab and vice versa as well as the delivery time from the sample location to the first lab or the delivery time from the sample location to the second lab may further be evaluated.

The route plan for the sample in or planned or expected or estimated to be in the first lab and the route plan for the sample in or planned or expected or estimated to be in the second lab may be estimated in advance. Thereby, a better estimate of the total handling time and, hence, processing time can be obtained. The route plan of the sample in a lab may depend on the route plan of the tubes in or planned or expected or estimated to be in said lab.

In a further embodiment, the selecting the processing lab may be done by using the state of the labs of the first plurality of labs. The selecting of the processing lab may include inquiring, in particular automatically inquiring, whether at least one lab of the first plurality of labs, comprises an instrument being able to carry out one or more of the tests. That is, it may be inquired whether the instruments of the labs of the first plurality of labs are working properly or are planned or expected or estimated to be working properly. In the affirmative, one lab among the first plurality of labs able to carry out the tests may be selected. In the selection of the processing, delivery times, processing times, and/or handling time may be used as described above. If none of the labs of the first plurality of labs is able to carry out one or more of the tests, the tests may be split in two subsets of tests, and selecting may comprise selecting a first lab, the first lab being able to carry out the first subset of the tests, and selecting a second lab, the second lab being able to carry out the second subset of tests may be determined. Subsequently, the sample may be moved to the first lab with the instruction to further move it to the second lab. Alternatively, the first computing device may notify a user that no lab of the first plurality of labs is able to carry out the tests to be performed on the sample.

For instance, the expected handling time and/or the expected processing time could be inferred by using historical data, statistics or using models such as simulations or machine learning models.

The determining, inquiring, evaluating and selecting may be done by the first computing device.

In a specific embodiment, the selecting by evaluating lab data may include applying machine learning algorithm to the lab data in order to optimise the selection of the processing laboratory and/or specifically carry out the selecting step without any intervention of another entity, such as a user or an agent or the like. A machine learning unit or device of the first computing device may be configured to perform the selecting of the processing lab by using a machine learning algorithm. The input data of said algorithm may be based on, comprise or consist of the lab data and/or the test data. The machine learning algorithm may be trained by using training data. The training data may comprise a plurality of data sets, each data set may comprise similar or the same information as the lab data.

Advantageously, by applying machine learning the selection of a processing lab may even further be improved. Thereby, delivery time of a sample and processing time of the sample may be reduced. By applying machine learning, it may be recognised by the first computing device which laboratories, in particular laboratory instruments, are, with a certain likelihood, frequently used and/or available most of the time and/or process samples in a predetermined time window. Further, it may be recognised by the first computing device which laboratories within a laboratory environment are able to perform a certain processing step within a predetermined time window for processing a sample and which lie within a certain delivery time and distance range. By machine learning, bottlenecks in handling and/or processing samples can be recognised, for example based on traffic data associated to the processing laboratories. The traffic data may comprise the workload of the processing laboratories and/or average delivery times and/or distances. Thus, the processing of a sample can be rendered even more efficient. Furthermore, it can be assured that the usage of lab resources is optimal and less lab resources are wasted.

In some embodiments, the method may comprise the step of instructing, bythe first computing device, the agent, a laboratory instrument and/or an automated laboratory instrument located at the sample location to prepare the sample tube by aliquoting a primary sample tube. In particular, this step may be carried out before the step of instructing the agent to initiate a transfer of the biological sample. This way, the stability of the sample against mechanical stresses that may arise from transportation is increased, as the sample tube is a secondary tube obtained by aliquoting the primary tube and, thus, does not comprise different phases. Hence, the reliability of the tests carried out on the sample at the processing lab is increased.

A second aspect of the present invention relates to a computer-implemented method, the method comprising at least the steps of:
- providing, by a second computing device, sample location data to a first computing device, the location data comprising information indicative of a location of a biological sample;
- obtaining, by the second computing device, transferring instructions from the first computing device, the transferring instructions instructing the second computing device to initiate a transfer of the biological sample from the sample location to a processing laboratory location; and
- initiating, by the second computing device, the transfer of the biological sample from the sample location to the processing laboratory location;
- wherein the processing laboratory location is a location of the processing laboratory.

Advantageously, according to the aforesaid aspect, a sample to be processed may be efficiently routed to a laboratory to handle the sample. In particular, transportation times may be reduced. Thus, the sample may be processed more efficiently.

In particular, the aforementioned aspect of the present invention also allows for reducing processing time, including transportation time of a sample, and/or transportation distance of a sample between laboratories of a plurality of laboratories, for example within a laboratory environment. The workload of the laboratories of the plurality of laboratories can be balanced. The processing time may be tuned with predetermined constraints comprising, for example, the time window within which a sample provides reliable results. Furthermore, the transportation time can be tuned with predetermined time constraints of the sample and of the tests to be carried out on the sample. The time constraints may comprise the time window within which the sample provides reliable results or the minimum amount of time that shall occur between a pre-analytical step, such as centrifuging, mixing with a reagent, etc., and the tests. Also, transportation distance may be tuned such that potential risks of contamination, for example due to environmental factors, may be reduced. A sample may be routed, in particular transferred or transported, from lab site to lab site within a lab environment according to the current or future state of the lab sites.

Features, embodiments, examples and advantages involved by the method according to the preceding aspect of the invention as described above apply to the current aspect as well.

The method of the second aspect may in particular be for local or lab-site or lab-wise routing and processing of the sample.

The providing sample location data may comprise sending data to the first computing device which may access said data in response to the sending. Alternatively, or in conjunction with the above, the providing may comprise making said data available to the first computing device. For instance, a second computing device may notify the first computing device that the data are available and allow the first computing device to access said data. This may for example be done by sending a link to the data to the first computing device.

The initiating of the transfer of the biological sample may comprise instructing a handling device to do some action that starts the transferring, the handling device being for example a robotic arm to move the sample to a rack for being transferred. The initiating may comprise providing transferring data or transferring information to a human operator. The initiating of the transfer of the biological sample may also be specified as already described above.

The method of the above-mentioned aspect may be performed in the following order: In a first step, the sample location data may be provided. In a next step, the transferring instructions are obtained. In a next step, in particular a final step, the sample transfer may be initiated.

In particular, the transferring instructions may be obtained by the second computing device from the first computing device. Alternatively, or in conjunction with the above, the first computing device may be configured to select, by evaluating laboratory data, the processing laboratory from a first plurality of laboratories, wherein the processing laboratory comprises one or more laboratory instruments, the one or more laboratory instruments being configured to carry out the one or more clinical tests to be performed on the biological sample. More particularly, the first computing device is the first computing device according to the first aspect of the present invention and may comprise any of the features thereof, as described in the present disclosure. In particular, the laboratory data are the laboratory data according to the first aspect of the present invention and may comprise any of the features thereof, as described in the present disclosure.

For example, the first computing device may be configured to select the processing lab based on one or more constraints and/or requirements as already described above.

In particular, the laboratory data may be the laboratory data according to the first aspect of the present invention and may comprise any of the features thereof, as described in the present disclosure.

For instance, the processing laboratory is selected from a plurality of laboratories by the first computing device by evaluating laboratory data, wherein the laboratory data comprise, for each of the first plurality of laboratories, information indicative of:
a respective distance associated to said each laboratory, and/or
   a respective delivery time associated to said each laboratory, and/or
a respective processing time associated to said each laboratory, and/or
a respective state of said each laboratory, and/or
a respective workload of said each laboratory and/or
   whether said laboratory is able to carry out the one or more clinical tests to be performed on the biological sample;
wherein:
   the respective distance associated to said each laboratory is a distance between the sample location and a laboratory location of said each laboratory;
   the respective delivery time associated to said each laboratory is an amount of time needed to deliver the biological sample from the sample location to the laboratory location of said each laboratory or an estimate and/or a prediction of the amount of time needed to deliver the biological sample from the sample location to the laboratory location of said each laboratory; and
   the respective processing time associated to said each laboratory is an amount of time needed to carry out the tests specified by the test data by using said each laboratory or the respective processing time associated to said each laboratory is an estimate and/or a prediction of the amount of time needed to carry out the tests specified by the test data by using said each laboratory.

Advantageously, the selection of a processing laboratory by evaluating said lab data, by the first computing device, allows for shortening processing times of samples. Clinical tests performed or carried out on a sample may be carried out more efficiently. Less lab-resources may be used or wasted.

In particular, by obtaining the lab data, the first computing device may efficiently select the optimal processing lab with respect to the respective processing time and/or distance and/or delivery time, or the processing lab chosen based on one or more predetermined constraints. For example, the delivery time may be tuned with respect to the time window within which reliable results of the samples to be processed are provided. This may even further reduce the processing time of a sample since the lab data are evaluated, in particular analysed or optimised, with respect to in particular distance and/or delivery time from the sample location to the processing lab and/or processing time of the processing lab.

In a specific embodiment, the method may further comprise the step of: providing, by the second computing device, test data to the first computing device, the test data comprising information indicative of one or more clinical tests to be performed on the biological sample.

Additionally or alternatively, the method may further comprise the step of: providing, by the second computing device, processing data to the first computing device, the processing data comprising information indicative of one or more processing steps that can be carried out at the sample location, wherein the one or more processing steps are determined based on the processing data. In particular, the processing data may comprise information as specified above.

A set of laboratory instruments may be located at the sample location. Information indicative of one or more processing steps that can be performed on the biological sample may comprise information specifying the state and/or the configuration of the set of lab instruments located at the sample location. In particular, the second computing device may be in data communication with the instruments of the set of lab instruments. More particularly, the second computing device may control the set of lab instruments.

The second computing device may be in data communication with the first computing device.

In a specific embodiment, the method may further comprise the steps of:
- obtaining, by the second computing device, processing instructions from the first computing device, the processing instructions instructing the second computing device to initiate carrying out a first processing step on the biological sample; and
- initiating, by the second computing device, the carrying out of the first processing step on the biological sample at the sample location, the first processing step being determined, based on processing laboratory data, by the first computing device, wherein the processing laboratory data comprises information indicative of a state and/or a configuration of one or more laboratory instruments of the processing laboratory.

Advantageously, according to this embodiment, a sample may be processed more efficiently, i.e. with reduced usage of laboratory resources since the processing steps to be performed on the sample are determined by the first computing device. In particular, the processing of a sample can be rendered even faster and less resource-consuming, i.e. without wasting lab capacities. Idle times of laboratories, in particular laboratory instruments, can be reduced further. A sample may be handled or processed iteratively until all processing steps, for example included in one or more tests to be carried out on the sample, are completed. Sample processing can be done more efficiently and autonomously further lowering the risk of spoiled samples and/or unreliable results.

The initiating of the carrying out of the first processing step may be carried out by carrying out said processing step. The initiating of the carrying out of the first processing step may comprise instructing a handling device to do some action that starts the first processing step. For instance, if the first processing step is centrifugation, the handling device may for example comprise or be a robotic arm that moves the sample to a centrifuge for centrifugation. The initiating of the carrying out of the first processing step may comprise providing the processing instructions to a human operator. For example, if the first processing step is centrifugation, the human operator may put the sample on the centrifuge for centrifugation.

In addition to the preceding embodiment, the step of initiating the carrying out of the first processing step on the biological sample at the sample location comprises instructing one or more laboratory instruments to carry out the first processing step. In particular, the one or more laboratory instruments are located at the sample location. Alternatively, or in conjunction with the above, the one or more laboratory instruments may be comprised in an automated laboratory system, e.g. located at the sample location. In particular, the second computing device may be operatively connected to the laboratory instruments and/or may be comprised in the automated laboratory system. The method of this aspect and the corresponding embodiments and the method of the preceding aspect and the corresponding embodiments may be performed at the same time or subsequently. The methods may be performed by a time shift. The methods may be performed by a system as described below.

A third aspect of the present invention relates to a computer program product, in particular stored in a computer-readable storage medium and/or in a data carrier and/or provided by a data stream, comprising instructions which, when the program is executed by a processor, cause the processor to carry out one or both of the methods according to the above-described aspects or corresponding embodiments.

The first computing device may comprise a computer program product comprising instructions which, when the program is executed by the first computing device, cause the first computing device to carry out the method according to the first aspect of the invention. The second computing device may comprise a computer program product comprising instructions which, when the program is executed by the second computing device, cause the second computing device to carry out the method according to the second aspect of the invention.

A fourth aspect of the invention relates to a data processing system. The data processing system comprises a processor configured to carry out the methods described herein. In particular, the first computing device may be a data processing system comprising a processor configured to carry out the method according to the first aspect of the invention. The second computing device may be a data processing system comprising a processor configured to carry out the method according to the second aspect of the invention.

A fifth aspect of the invention relates to a computer-readable medium. The computer-readable medium comprises instructions which, when executed by a computer, cause the computer to carry out the methods described herein.

A sixth aspect of the present invention relates to a system, in particular for routing and processing a biological sample, comprising:
- a first plurality of laboratories, wherein each laboratory of the first plurality of laboratories comprises one or more laboratory instruments;
- a first computing device, the first computing device comprising:
   a storage unit;
   an obtaining unit configured to obtain sample location data, the sample location data comprising information indicative of a sample location of the biological sample and to obtain test data, the test data comprising information indicative of one or more clinical tests to be performed on the biological sample;
   a selecting unit configured to select by evaluating laboratory data a processing laboratory from the first plurality of laboratories, wherein the processing laboratory is located at a processing laboratory location and the processing laboratory comprises one or more laboratory instruments, the one or more laboratory instruments being configured to carry out the one or more clinical tests to be performed on the biological sample; and
   an instructing unit configured to instruct initiation of a transfer of the biological sample from the sample location to the processing laboratory location; and
- at least one second computing device, the at least one second computing device comprising:
   a storage unit;
   a providing unit configured to provide the sample location data to the first computing device;
   an obtaining unit configured to obtain from the first computing device transferring instructions, the transferring instructions instructing the at least one second computing device to initiate the transfer of the biological sample from the sample location to a selected processing laboratory location; and
   an initiating unit configured to initiate the transfer of the biological sample from the sample location to the selected processing laboratory location.

Advantageously, the system according to the aforesaid aspect allows for reducing processing time of a sample. Thereby, the processing of a sample can be rendered more efficient. That is, laboratory resources can be used more efficiently. In other words, less laboratory resources are wasted. Furthermore, the processing of multiple samples can be streamlined. In particular, the system also allows for reducing processing time, including transportation time of a sample, and/or transportation distance of a sample between laboratories of a plurality of laboratories, for example within a laboratory environment. The workload of the laboratories of the plurality of laboratories can be balanced. The processing time may be tuned with predetermined constraints comprising, for example, the time window within which a sample provides reliable results. Furthermore, the transportation time can be tuned with predetermined time constraints of the sample and of the tests to be carried out on the sample. The time constraints may comprise the time window within which the sample provides reliable results or the minimum amount of time that shall occur between a pre-analytical step, such as centrifuging, mixing with a reagent, etc., and the tests. Also, transportation distance may be tuned such that potential risks of contamination, for example due to environmental factors, may be reduced. A sample may be routed, in particular transferred or transported, from lab site to lab site within a lab environment according to the current or future state of the lab sites. In addition to the above, the advantages described with respect to the first aspect equally apply to this aspect.

Features, embodiments, examples and advantages as described with respect to the methods according to one of the preceding aspects of the invention apply to the current aspect as well.

The first plurality of labs may be distributed over several sites. The first plurality of labs may also be contained in one lab complex located at one lab site. The first plurality of labs may be contained in a laboratory environment.

The first computing device may comprise or be a cloud computing platform or system. It may comprise or be a cluster computer or a distributed computing system. The first computing device may be located at one of the first plurality of labs. The first computing device may be configured to carry out the method of the first aspect of the present invention. Each unit of the first computing device may be configured to perform one step of said method. Each of the units of the first computing device may be a standalone computing device having at least one processor, such as a desktop PC, workstation, laptop or tablet PC, whereby the units are connected to each other and in data communication with each other. Each unit may also correspond to a cluster of a cluster computer. Each unit of the first computing device may be associated with one or more processors of the computing device. The storage unit of the first computing device is a unit configured to store data therein or thereon. It may comprise or be a magnetic storing device, such as a hard drive disk, and/or a solid-state disk and/or a cloud storage.

The at least one second computing device may comprise or be a cluster computer. The at least one second computing device may comprise or be a desktop PC, a workstation, a laptop or a tablet PC. The at least one second computing device may be located at one of the first plurality of labs. The at least one second computing device may comprise a plurality of second computing devices. Each lab of the first plurality of labs may comprise one of the plurality of second computing devices. The at least one second computing device and the first computing device may be operatively connected and in data communication with each other. The at least one second computing device may be configured to carry out the method of the second aspect of the present invention. Each unit of the at least one second computing device may be configured to perform one step of said method. Each of the units of the at least one second computing device may be a standalone computing device having at least one processor, such as a desktop PC, workstation, laptop or tablet PC, whereby the units are connected to each other and in data communication with each other. Each unit may also correspond to a cluster of a cluster computer. Each unit of the at least one second computing device may be associated with one or more processors of the at least one second computing device. The storage unit of the at least one second computing device is a unit configured to store data therein or thereon. It may comprise or be a magnetic storing device, such as a hard drive disk, and/or a solid-state disk and/or a cloud storage.

In a specific embodiment, the at least one second computing device may be in communication with the first computing device. Data may be exchanged between the first computing device and the at least one second computing device.

Additionally or alternatively, each of the first plurality of laboratories may be in communication with the first computing device. The first computing device may be configured to send instructions to carry out one or more clinical tests and/or data to each of the first plurality of laboratories.

Additionally or alternatively, the laboratories of the first plurality of laboratories may be in communication with each other. The communication may be provided by means of a network. Data may be exchanged between the labs of the first plurality of labs.

In a specific embodiment, the one or more laboratory instruments of at least one laboratory of the first plurality of laboratories may be comprised in an automated laboratory system of the at least one laboratory.

Additionally or alternatively, the first computing device may be a cloud computing device.

Additionally or alternatively, the first computing device and the at least one second computing device form a distributed computing system.

Additionally or alternatively, the system may further comprise a machine-learning unit or device to optimise the selection of a processing laboratory of the first plurality of laboratories based on the laboratory data. The optimisation may further be based on processing laboratory data. The machine learning unit may comprise a neural network, e.g. a trained neural network, that processes the lab data, and optionally the processing lab data, to select, in particular optimise the selection of, the processing lab. The input data for the neural network may be based on, comprise, or consist of the lab data and/or the test data, and optionally on the processing lab data.

Advantageously, the selection of a processing laboratory to which a sample is to be transferred to perform one or more clinical tests or a step of said tests may further be improved. Thereby, the processing of samples may be rendered even more efficient. Wasting lab resources may be reduced further.

In particular, by applying machine learning by the machine learning unit, the selection of a processing lab may even further be improved. Thereby, delivery time of a sample and processing time of the sample may be reduced. By applying machine learning, it may be recognised by the first computing device in an autonomous fashion, which laboratories, in particular laboratory instruments, are, with a certain likelihood, frequently used and/or available most of the time and/or process samples in a predetermined time window. Further, it may be recognised by the first computing device in an autonomous fashion, which laboratories within a laboratory environment are able to perform a certain processing step within a predetermined time window for processing a sample and which lie within a certain delivery time and distance range. By machine learning, bottlenecks in handling and/or processing samples can be recognised, for example based on traffic data associated to the processing laboratories. The traffic data may comprise the workload of the processing laboratories and/or average delivery times and/or distances. Thus, the processing of a sample can be rendered even more efficient. Furthermore, less lab resources are wasted.

Additionally or alternatively, at least one laboratory of the first plurality of laboratories comprises the at least one second computing device.

In an embodiment, the system may further comprise sample-transportation-means. The sample-transportation means may include or be means for delivering, transferring or transporting at least one sample from a location to another location, such as a conveyor band and/or means for pneumatic delivery of sample tubes.

The selected processing laboratory may be selected as already described above with respect to one of the methods according to one of the aspects of the present invention.

### Brief Description of the Drawings

Details of exemplary embodiments are set forth below with reference to the exemplary drawings. Other features will be apparent from the description, the drawings, and from the claims. It should be understood, however, that even though embodiments are separately described, single features of different embodiments may be combined to further embodiments.
**Figure 1** shows a schematic representation of an embodiment of a system according to an aspect of the present invention; and
**Figure 2** shows a flow diagram including exemplary steps performed by the first computing device and by the second computing device.

### Detailed Description

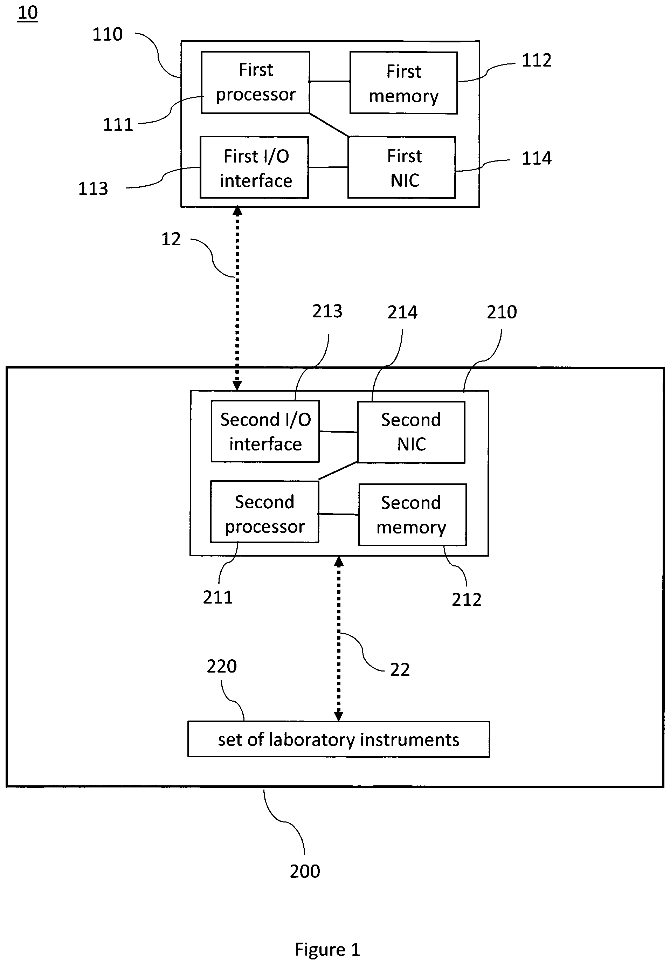

**Figure 1** shows a schematic representation of an embodiment of a system 10 according to an aspect of the present invention. Specifically, figure 1 shows a schematic representation of an exemplary first computing device 110 and of an exemplary second computing device 210.

In the embodiment shown in figure 1, the first computing device 110 is a cluster computer located at a location different from a laboratory at which a particular sample to be processed is located, defining the sample location 200. The first computing device 110 may also be a desktop PC, a sever, a distributed computing system or a cloud computing system.

The first computing device 110 comprises a first processor 111, a first memory 112 or storage unit, a first I/O interface 113 and a first interface controller 114 (NIC). The first processor 111 is connected to the first memory 112 and the first NIC 114. The first NIC 114 is connected to the first I/O interface 113. The first I/O interface 113 is configured to send output of the first computing device 110 to or receive input from at least one second computing device 210 by means of a protocol suite to exchange data 12. In figure 1, only one second computing device 210 is shown. The first processor 111 is configured to carry out the method according to the first aspect described above. The first memory 112 may comprise a primary memory and a secondary memory (not shown). In particular, the secondary memory stores a computer program (comprising instructions which when executed by the processor 111 cause the first computing device 110 to carry out the method according to the first aspect described above.

The second computing device 210 shown in figure 1 is located at a location different from the first computing device 110. The second computing device 210 is located at the sample location 200 or laboratory where the sample to be processed is located. The second computing device 210 may be a desktop PC, a workstation, a cluster computer or a server. The second computing device 210 comprises a second processor 211, a second memory 212 or storage unit, a second I/O interface 213 and a second NIC 214. The second NIC 214 is connected to the second processor 211 and the second I/O interface 213. The second processor 211 is also connected to the second memory 212. The second processor 211 is configured to carry out the method according to the second aspect described above. The second memory 212 may comprise a primary memory and a secondary memory (not shown). In particular, the secondary memory stores a computer program comprising instructions which, when executed by the processor 211, cause the second computing device 210 to carry out the method according to the second aspect described above.

The first memory 112 as well as the second memory 212 may comprise or be a RAM and/or a magnetic hard drive and/or a solid-state storage and/or a cloud storage.

The second computing device 210 may output instructions to or receive data from a set of laboratory instruments 222 which are located at the laboratory of the sample location 200. The input and output are transmitted between the second computing device 210 and the set of laboratory instruments 222 by means of the protocol suite to exchange data 22.

Via the protocol suite 12, the first computing device 110 may transmit instructions to the second computing device 210 to perform one or more clinical tests, in particular one or more processing steps, on a sample located at the sample location 200. The second computing device 210 receives the instructions and initiates the performing of the one or more clinical tests by instructing the set of laboratory instruments 222 at the sample location 200 accordingly. The set of lab instruments 222 may for instance comprise or be an automated laboratory system that is in data communication with the second computing device 210.

The first and second computing devices 110, 210 may comprise more than one processor 111, 211 for task parallelization.

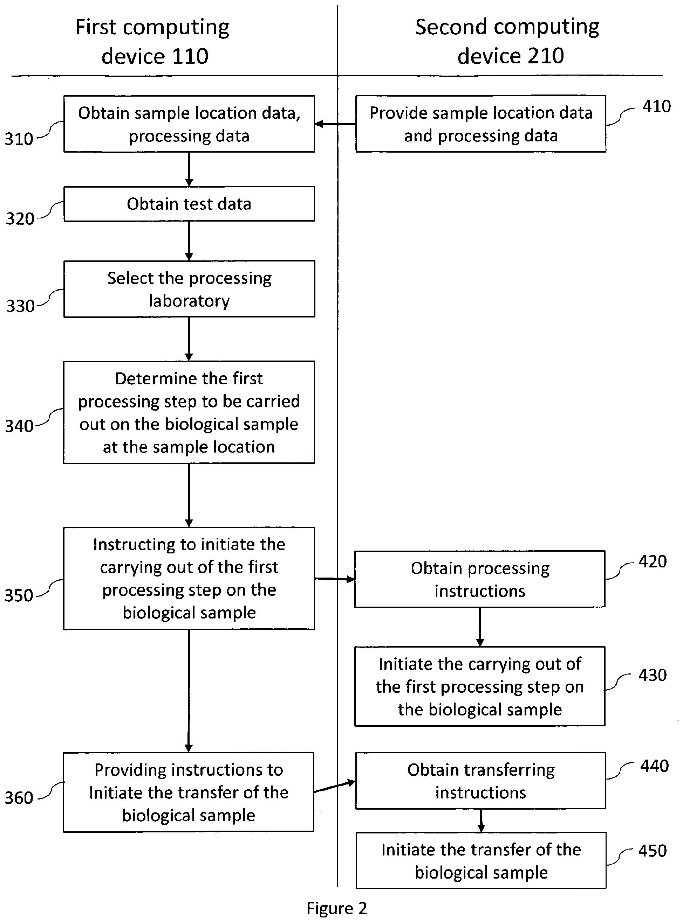

**Figure 2** shows a flow diagram including exemplary steps performed by the first computing device 110 and by the second computing device 210.

Figure 2 in particular shows how the methods according to above-described aspects of the present invention may be connected and performed together by the system 10, specifically the first computing device 110 and the second computing device 210.

The first computing device 110 obtains, in a step 310, sample location data and processing data for a sample to be processed, the sample location data and processing data being provided by the second computing device 210, for example via the protocol suite 12 in figure 1, in a step 410. The sample location data comprise data such as location identification data of the sample to be processed. These data include for example GPS coordinates of the lab in which the sample is located or the identification number of the lab in which the sample is located. The processing data comprise for example information indicative of the processing steps which can be performed in this lab. In particular, the processing data include information indicative of the availability and/or configuration and/or workload, of the lab instruments 222 located at the sample location 200.

In a next step 320, the first computing device 110 obtains test data are. The test data include information indicative of the clinical tests to be performed on the sample to be processed. The test data may be obtained by manual input by a user e.g. using a keyboard and typing in the data and/or using a mobile storage device, such as a USB drive, CD, or the like. Alternatively, or in conjunction with the above, the test data may be obtained by querying a database. This database may be stored in the first computing device 110. In addition or as an alternative, this database may partially or completely be a network connected drive and/or part of a cloud storage and accessed via a network. The test data may be referenced to the sample to be processed and accessed by searching for the sample in the database. The test data may also be provided to the first computing device 110 by the second computing device 210.

In a next step 330, the first computing device 110 selects a processing lab out of a first plurality of laboratories is selected. The selection is done by evaluating laboratory data.

In particular, the first computing device 110 receives from each lab (not shown) of the first plurality of labs, a respective portion of the lab data. For each lab, the respective portion of lab data comprises information indicative of whether the lab. is able to carry the clinical tests to be performed on the biological sample. For each lab which is able to carry out the one or more clinical tests to be performed on the biological sample, the respective portion of lab data comprises also information indicative of the handling time associated to this lab. The first computing device 110 uses the information indicative of whether the labs of the first plurality of labs that are able to carry the clinical tests to be performed on the biological sample to identify the second plurality of labs that are able to carry out said tests. Hence, the the first computing device 110 may retrieve, for each lab of the second plurality of labs, the respective distance and use the distance together with the respective handling time to estimate, for each lab of the second plurality of labs, the respective processing time. The lab of the second plurality of labs associated with the smallest processing time is selected as processing lab.

Alternatively with the above, the laboratory data may for example include the delivery time and/or distance to each laboratory of the first plurality of labs and/or the workload of each lab of the first plurality of labs. The first computing system 110 may evaluate the lab data such that it finds the optimal processing laboratory with respect to said lab data. The term "optimal" does not necessarily imply the processing lab with the minimal distance and/or delivery time from the sample location 200. It may also refer to the optimal processing lab with respect to some predetermined constraint or constraints such as the processing lab within a radius around the sample location 200 of a given or predetermined amount or number of kilometers, such as 20 kilometers or 10 kilometers and a given or predetermined delivery time of at most 20 minutes or at most 15 minutes or between 5 and 10 minutes.

In addition or as an alternative, the optimal processing lab may be the lab with a preferred or predetermined set of lab instruments 222.

The lab data may also be evaluated by the first computing device 110 by using a machine learning device or unit. Thus, the selection of the processing lab may be improved. For example, the machine learning device or unit may select the processing lab by using a trained neural network which processes the lab data to select the processing lab.

In a next step 340, the first computing device 110 determines at least a first processing step to be performed on the sample in the lab at the sample location 200. This determination may be based on the lab data and/or processing lab data of the selected processing lab, wherein the processing laboratory data comprise information indicative of a state and/or a configuration of the one or more laboratory instruments of the processing laboratory. In other words, the first computing device 110 may determine which processing steps can be performed at the processing lab and which can be performed at the sample location 200. For instance, the first processing step may be a centrifugation or a gas blood test, the latter test having a relatively short stability time. If the first processing stap is centrifugation, in particular, the first computing device 110 may assess, by using the test data, whether the sample is to be centrifuged or not. If the sample is to be centrifuged the first computing device 110 accesses the lab data to assess whether centrifugation can be carried out at the sample location. If this is the case, the centrifugation is the first processing step to be performed at the sample location 200.

In a next step 350, the first computing device 110 provides instructions to the second computing device 210, being located at the sample location 200, to carry out the determined first processing step at the sample location 200. In a step 420, on the side of the second computing device 210, the second computing device 210 obtains the instructions provided by the first computing device 110 and initiates, in a step 430, the carrying out of the first processing step on the sample to be processed at the sample location 200.

Subsequently or simultaneously, in a step 360, the first computing device 110 provides instructions to the second computing device 210 to initiate transferring of the sample to be processed from the sample location 200 to the selected processing lab to perform the remaining processing steps. In a step 440, , the second computing device 210 obtains or receives the transferring instructions by the first computing device 110 and initiates, in a step 450, the transfer of the sample to the selected processing laboratory.

In conclusion, the inventors have recognized that the methods and the system as described herein provide the advantages that sample processing can be facilitated and performed in a time and resource saving manner. Processing times may be reduced or shortened and laboratory resources can be used more efficiently.

### Reference Numerals

- 10: system
- 110: first computing device
- 200: sample location
- 210: second computing device
- 111, 211: processor
- 112, 212: memory
- 113, 213: I/O interface
- 114, 214: network interface controller (NIC)
- 222: lab instruments
- 12, 22: protocol suite to exchange data

## Claims

1. A computer-implemented method, the method comprising at least the steps of:
- obtaining (310), by a first computing device (110), sample location data, the sample location data comprising information indicative of a sample location (200) of a biological sample;
- obtaining (320), by the first computing device (110), test data, the test data comprising information indicative of one or more clinical tests to be performed on the biological sample;
- selecting (330) by evaluating laboratory data, by the first computing device (110), a processing laboratory from a first plurality of laboratories, wherein the processing laboratory is located at a processing laboratory location and the processing laboratory comprises one or more laboratory instruments (222), the one or more laboratory instruments (222) being configured to carry out the one or more clinical tests to be performed on the biological sample; and
- instructing (360), by the first computing device (110), an agent to initiate a transfer of the biological sample from the sample location (200) to the processing laboratory location.

2. The method according to claim 1, wherein the method further comprises:
obtaining, by the first computing device (110), the laboratory data, the laboratory data comprising, for each laboratory of the first plurality of laboratories, information indicative of:
a respective distance associated to said each laboratory, and/or
a respective delivery time associated to said each laboratory, and/or
a respective processing time associated to said each laboratory, and/or
a respective state of said each laboratory, and/or
a respective workload of said each laboratory, and/or
whether said laboratory is able to carry out the one or more clinical tests to be performed on the biological sample;
wherein:
the respective distance associated to said each laboratory is a distance between the sample location and a laboratory location of said each laboratory;
the respective delivery time associated to said each laboratory is an amount of time needed to deliver the biological sample from the sample location to the laboratory location of said each laboratory or an estimate and/or prediction of the time needed to deliver the biological sample from the sample location to the laboratory location of said each laboratory; and
the respective processing time associated to said each laboratory is an amount of time needed to carry out the tests specified by the test data by using said each laboratory or an estimate and/or a prediction of the amount of time needed to carry out the tests specified by the test data by using said each laboratory.

3. The method according to claim 2, wherein the step of selecting the processing laboratory comprises determining at least a first laboratory of the first plurality of laboratories so that the first laboratory fulfils one or more instruments requirements, and/or one or more distance requirements and/or one or more delivery time requirements, and/or one or more processing time requirements.

4. The method according to claim 3,
(i) wherein the one or more distance requirements comprise the requirement that the distance associated to the first laboratory is lower than or equal to a distance threshold, and/or wherein the one or more distance requirements comprise the requirement that, for each laboratory of a second plurality of laboratories, the distance associated to the first laboratory is smaller than or equal to the distance associated to said each laboratory; and/or
(ii) wherein the one or more instruments requirements comprise the requirement that the set of laboratory instruments comprised in the first laboratory is configured to carry out the one or more clinical tests; and/or
(iii) wherein the one or more delivery time requirements comprise the requirement that the delivery time associated to the first laboratory is lower than or equal to a time delivery threshold, and/or wherein the one or more delivery time requirements comprise the requirement that, for each laboratory of a second plurality of laboratories, the delivery time associated to the first laboratory is smaller than or equal to the delivery time associated to said each laboratory; and/or
(iv) wherein the one or more processing time requirements comprise the requirement that the processing time associated to the first laboratory is lower than or equal to a time handling threshold, and/or wherein the one or more processing time requirements comprise the requirement that, for each laboratory of the second plurality of laboratories, the processing time associated to the first laboratory is smaller than or equal to the processing time associated to said each laboratory; and/or
(v) wherein, in order for the first computing device (110) to select the first laboratory as processing laboratory, the first laboratory is to fulfil the one or more instruments requirements, and/or the one or more distance requirements, and/or the one or more delivery time requirements, and/or the one or more processing time requirements, wherein the first plurality of laboratories comprises the second plurality of laboratories.

5. The method according to any one of claims 1 to 4, further comprising the steps of:
determining (340), by the first computing device (110), a first processing step to be carried out on the biological sample at the sample location (200);
instructing (350), by the first computing device (110), the agent, in particular a second computing device (210), to initiate the carrying out of the first processing step on the biological sample at the sample location (200),
wherein the step of determining the first processing step is based on processing laboratory data and/or processing data,
wherein the processing laboratory data comprise information indicative of a state and/or a configuration of the one or more laboratory instruments of the processing laboratory, and the processing data comprise information indicative of one or more processing steps that can be carried out at the sample location (200).

6. A computer-implemented method, the method comprising at least the steps of:
- providing (410), by a second computing device (210), sample location data to a first computing device (110), the location data comprising information indicative of a location of a biological sample;
- obtaining (440), by the second computing device (210), transferring instructions from the first computing device (110), the transferring instructions instructing the second computing device (210) to initiate a transfer of the biological sample from the sample location (200) to a processing laboratory location; and
- initiating (450), by the second computing device (210), the transfer of the biological sample from the sample location (200) to the processing laboratory location;
- wherein the processing laboratory location is a location of the processing laboratory.

7. The method according to claim 6,
wherein the processing laboratory is selected from a first plurality of laboratories by the first computing device (110) by evaluating laboratory data, wherein the laboratory data comprise, for each of the first plurality of laboratories, information indicative of:
a respective distance associated to said each laboratory, and/or
a respective delivery time associated to said each laboratory, and/or
a respective processing time associated to said each laboratory, and/or
a respective state of said each laboratory, and/or
a respective workload of said each laboratory, and/or
whether said laboratory is able to carry out the one or more clinical tests to be performed on the biological sample;
wherein:
the respective distance associated to said each laboratory is a distance between the sample location and a laboratory location of said each laboratory;
the respective delivery time associated to said each laboratory is a time needed to deliver the biological sample from the sample location to the laboratory location of said each laboratory; and
the respective processing time associated to said each laboratory is an amount of time needed to carry out the tests specified by the test data by using said each laboratory or an estimate and/or a prediction of the amount of time needed to carry out the tests specified by the test data by using said each laboratory.

8. The method according to any one of claims 6 and 7,
(i) further comprising the step of:
providing, by the second computing device (210), test data to the first computing device (110), the test data comprising information indicative of one or more clinical tests to be performed on the biological sample; and/or
(ii) further comprising the step of:
Providing (410), by the second computing device (210), processing data to the first computing device (110), the processing data comprising information indicative of one or more processing steps that can be carried out at the sample location (200), wherein the one or more processing steps are determined based on the processing data.

9. The method according to any one of claims 6 to 8, the method further comprising the steps of:
- obtaining (420), by the second computing device (210), processing instructions from the first computing device (110), the processing instructions instructing the second computing device (210) to initiate carrying out a first processing step on the biological sample; and
- initiating (430), by the second computing device (210), the carrying out of the first processing step on the biological sample at the sample location (200), the first processing step being determined, based on processing laboratory data, by the first computing device (110), wherein the processing laboratory data comprises information indicative of a state and/or a configuration of one or more laboratory instruments (222) of the processing laboratory.

10. The method according to claim 9, wherein the step of initiating the carrying out of the first processing step on the biological sample at the sample location (200) comprises instructing one or more laboratory instruments (222) to carry out the first processing step, wherein the one or more laboratory instruments (222) are optionally comprised in an automated laboratory system.

11. A computer program product, in particular stored in a computer-readable storage medium and/or in a data carrier and/or provided by a data stream, comprising instructions which, when the program is executed by a processor, cause the processor to carry out the method according to any one of claims 1 to 6, and/or the method according to any one of claims 7 to 11.

12. A system (10), in particular for routing and processing a biological sample, comprising:
- a first plurality of laboratories, wherein each laboratory of the first plurality of laboratories comprises one or more laboratory instruments (222);
- a first computing device (110), the first computing device (110) comprising:
a storage unit (112);
an obtaining unit configured to obtain sample location data, the sample location data comprising information indicative of a sample location (200) of the biological sample and to obtain test data, the test data comprising information indicative of one or more clinical tests to be performed on the biological sample;
a selecting unit configured to select by evaluating laboratory data a processing laboratory from the first plurality of laboratories, wherein the processing laboratory is located at a processing laboratory location and the processing laboratory comprises one or more laboratory instruments, the one or more laboratory instruments being configured to carry out the one or more clinical tests to be performed on the biological sample; and
an instructing unit configured to instruct initiation of a transfer of the biological sample from the sample location (200) to the processing laboratory location; and
- at least one second computing device (210), the at least one second computing device (210) comprising:
a storage unit (212);
a providing unit configured to provide the sample location data to the first computing device (110);
an obtaining unit configured to obtain from the first computing device (110) transferring instructions, the transferring instructions instructing the at least one second computing device (210) to initiate the transfer of the biological sample from the sample location (200) to a selected processing laboratory location; and
an initiating unit configured to initiate the transfer of the biological sample from the sample location (200) to the selected processing laboratory location.

13. The system (10) according to claim 12, wherein the at least one second computing device (210) is in communication with the first computing device (110); and/or
wherein each of the first plurality of laboratories is in communication with the first computing device (110); and/or
wherein the laboratories of the first plurality of laboratories are in communication with each other;
wherein the communication is optionally provided by means of a network.

14. The system (10) according to any one of claims 12 and 13, wherein the one or more laboratory instruments (222) of at least one laboratory of the first plurality of laboratories are comprised in an automated laboratory system of the at least one laboratory; and/or
wherein the first computing device (110) is a cloud computing device; and/or
wherein the first computing device (110) and the at least one second computing device (210) form a distributed computing system; and/or
wherein the system (10) further comprises a machine-learning device to optimize the selection of a processing laboratory of the first plurality of laboratories based on the laboratory data, and optionally further based on processing laboratory data; and/or
wherein at least one laboratory of the first plurality of laboratories comprises the at least one second computing device (210).
